# EUROPÄISCHE PATENTSCHRIFT

(11) **EP 0 997 187 B1**
(45) Veröffentlichungstag und Bekanntmachung des Hinweises auf die Patenterteilung: **29.01.2003**
(21) Anmeldenummer: 99119504.1
(22) Anmeldetag: 01.10.1999
(51) Int. Cl.: B01J 8/18, B01J 8/24, C07C 17/156, C07C 19/045

(54) **Wirbelschicht-Reaktor zur Oxichlorierung von Ethylen, Sauerstoff und HCl**
Fluidised bed reactor for the oxychlorination of ethylene, oxygen and HCl
Réacteur à lit fluidisé pour l'oxychloruration d'éthylène, oxygène et HCl

(30) Priorität: 28.10.1998 DE 19849709
(43) Veröffentlichungstag der Anmeldung: 03.05.2000
(73) Patentinhaber: Uhde GmbH, 44141 Dortmund (DE)
(72) Erfinder: Schön, Hartmut, 61440 Oberursel (DE)
(74) Vertreter: Patentanwälte Meinke, Dabringhaus und Partner

(56) Entgegenhaltungen:
- GB-A- 1 391 430
- US-A- 2 500 519
- US-A- 2 931 711
- US-A- 3 679 373
- US-A- 3 991 096
- US-A- 4 197 418
- US-A- 4 526 759

## Beschreibung

Die Erfindung richtet sich auf einen Wirbelschicht-Reaktor zur Oxichlorierung von Ethylen, Sauerstoff und HCl mit einem aus mehreren Rohrpaketen bestehenden Wärmetauscher in der Wirbelschicht zur Abgabe der aufgrund exothermer Reaktionen entstehenden Wärme an einem Wärmeträger in den Rohrpaketen, insbesondere an Wasser/Dampf, wobei die Rohrpakete über eine Ringleitung mit Wasser beaufschlagt und der Dampf über eine Ringleitung abgenommen wird, wobei die Ringleitungen als Sammler oder Kammer unmittelbar auf den Reaktormantel aufgesetzt sind.

Bei der Oxichlorierung werden Ethylen, Sauerstoff und HCl in einem Wirbelschicht-Reaktor (Oxi-Reaktor) an einem kupferhaltigen Katalysator zu 1,2-Dichlorethan und Wasser umgesetzt. Die bei dieser Umsetzung entstehende Wärme wird vom Katalysator über ein im Reaktor befindliches Rohrsystem (bestehend aus mehreren Rohrpaketen) an Boiler Feed Water zur Dampferzeugung oder einen Wärmeträger abgegeben. Das BFW (der Wärmeträger) wird durch eine außerhalb des Reaktors liegende Ringleitung auf das Rohrsystem verteilt. Der entstehende Dampf (der aufgeheizte Wärmeträger) wird über eine ebenfalls außerhalb des Reaktors liegende Ringleitung gesammelt und abgeführt.

Bei der bekannten Ausführungsform bestehen u.a. folgende Nachteile:

Die beiden außen liegenden Ringleitungen haben je nach Anzahl der Rohrpakete im innen liegenden Rohrsystem eine Vielzahl von Verbindungsleitungen durch den Mantel zu den Rohrpaketen. In den Verbindungsleitungen für das Kühlwasser (den Wärmeträger) werden in zusätzlichen Flanschverbindungen Lochscheiben eingesetzt, um über den Druckverlust eine gleichmäßige Verteilung auf die einzelnen Rohrpakete zu erreichen. Die Begehbarkeit und Wartung der Ringleitungen wird über eine 360° Bühne erreicht. Eine Abbildung der Ringleitungen als Modell für die Rohrspannungsberechnungen ist sehr komplex und aufwendig. Der Oxi-Reaktor muß sehr sorgfältig isoliert werden, um Taupunktunterschreitung zu vermeiden. Durch die vielen Mantelstutzen für die Ringleitungsanschlüsse und die Bühnenkonsolen ist das schwierig und zeitraubend.

Aus dem Anlagebau sind Reaktoren unterschiedlicher Bauweise bekannt, die auch Ringleitungen zeigen, so ein Wärmetauscher nach der GB-1 391 430 oder ein Wirbelschichtreaktor nach US-2 931 711. Zum weiteren Stand der Technik seien noch die US-Patentschriften 2 500 519, 3 679 373, 3 991 096, 4 197 418 oder 4 526 759 erwähnt.

Aufgabe der Erfindung ist die Schaffung einer Lösung, mit der unter Vermeidung der oben beschriebenen Nachteile eine kompakte, leicht herzustellende, aber kostengünstige Lösung geschaffen wird, die die aufwendigen Bohrdurchführungen vermeidet, insbesondere auch die Berechnung von Ringleitungen erleichtert und eine Vielzahl von Manteldurchtritten entbehrlich macht.

Mit einer Vorrichtung der eingangs bezeichneten Art wird diese Aufgabe gemäß der Erfindung dadurch gelöst, daß die Verteil- bzw. Sammelkammern querschnittlich im wesentlichen kreisförmig ausgebildet sind und sowohl innen als auch außen auf den Reaktormantel aufgesetzt sind, wobei eine Hälfte dem Inneren des Reaktors und die andere Hälfte dem Äußeren des Reaktors zugeordnet ist.

Durch die Erfindung ist es möglich, die Vielzahl von Manteldurchtritten bzw. die Lochscheiben durch einfache Bohrungen zu ersetzen im innen liegenden Sammler, damit die Isolierung des Reaktors erheblich vereinfacht wird.

Eine besondere Ausgestaltung besteht zum Beispiel darin, daß die Bohrungen zum Anschluß der Rohrleitungen zur Definierung eines gewünschten Druckverlustes und damit zur Vergleichmäßigung der Beaufschlagungen der unterschiedlichen Rohrpakete als Drosselbohrungen aufgeführt sind.

Damit kann allein durch die entsprechende vorberechnete Wahl des Durchmessers auf die Druckverteilung im Sammler und den Rohrleitungen Einfluß genommen werden.

Die Erfindung ist nachstehend anhand der Zeichnung beispielsweise näher erläutert. Diese zeigt in
- Fig. 1: eine schematische vereinfachte Darstellung des Durchführungsbereiches der Rohrleitungen nach dem Stand der Technik mit außen liegenden Ringsammlern,
- Fig. 2: in vergleichsweiser Darstellung eine nicht-erfindungsgemäße Ausgestaltung
- Fig. 3 bis 5 und 7: unterschiedliche nicht-erfindungsgemäßen Gestaltungsvarianten in vereinfachten Schnittdarstellungen im Bereich des Reaktormantels.
- Fig. 6 und 8: unterschiedliche Gestaltungsvarianten der Erfindung in vereinfachten Schnittdarstellungen im Bereich des Reaktormantels.

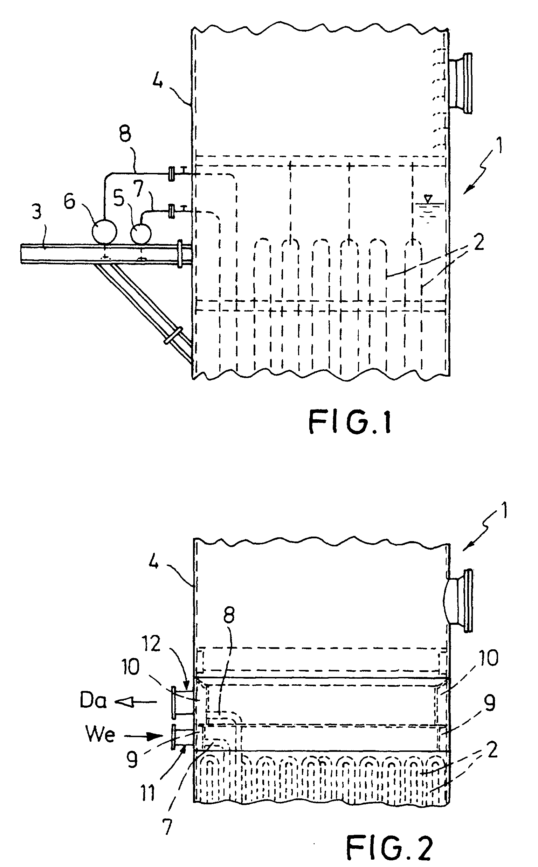

In Fig. 1 ist eine Lösung nach dem Stand der Technik dargestellt. Hier weist der allgemein mit 1 bezeichnete Reaktor eine Vielzahl von Rohrpaketen 2 als Wärmetauscher auf mit auf einer Konsole 3 außerhalb des Reaktormantels 4 angeordneten Ringleitungen 5 und 6 für beispielsweise das zulaufende Kühlwasser in der Ringleitung 5 und dem abgezogenen Dampf in der Ringleitung 6. Erkennbar müssen aber Zu- und Abflußleitungen 7 bzw. 8 individuell durch den Reaktormantel geführt werden und dem damit verbundenen rechnerischen, konstruktiven und fertigungstechnischen Aufwand.

In Fig. 2 ist in gleicher Darstellungsart ein vereinfachter Schnitt durch den entsprechenden Teil eines nicht-erfindungsgemäßen Reaktors, ebenfalls mit 1 bezeichnet, dargestellt. Die Rohrbündel 2 enden hier in zwei im Inneren an der Wand angebrachten, beispielsweise rechteckigen oder trapezförmigen Ringsammlern 9 und 10, wobei über den Ringsammler bzw. Verteiler 9 das Wärmetauschermedium eingebracht und beispielsweise über den Sammler 10 der Dampf abgezogen wird. Dazu durchsetzen lediglich im dargestellten Beispiel der Fig. 2 zwei Querstutzen 11 bzw. 12 den Reaktormantel 4. Die Zulaufleitungen 7 bzw. die Rückführleitungen 8 für den Dampf durchsetzen lediglich die Innenwand der Sammler 9 bzw. 10.

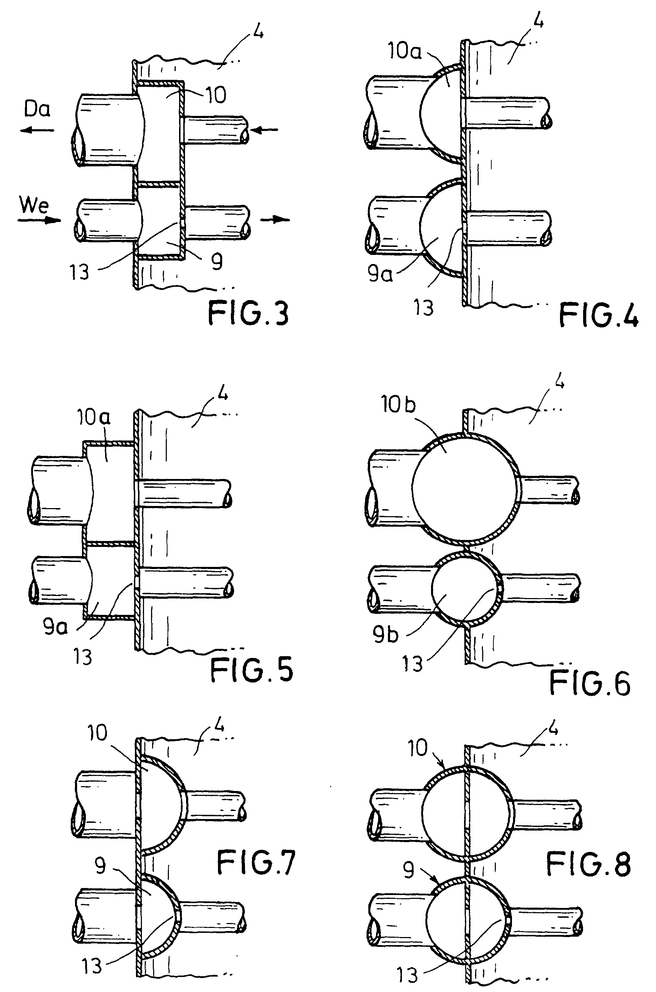

In den Fig. 3 bis 8 sind Ausgestaltungen dargestellt, was Form und Anbringung der Sammler 9 und 10 angeht. Erkennbar sind beispielsweise in den Durchtrittswänden bzw. auch dem Reaktormantel Drosselbohrungen, allgemein mit 13 bezeichnet, vorgesehen, um Druckunterschiede aufzubauen bzw. auszugleichen. Diese Bohrungen können je nach Positionierung relativ zum Zu- bzw. Ablaufstutzen vom Umfang variieren.

## Patentansprüche

1. Wirbelschicht-Reaktor (1) zur Oxichlorierung von Ethylen, Sauerstoff und HCl mit einem aus mehreren Rohrpaketen (2) bestehenden Wärmetauscher in der Wirbelschicht zur Abgabe der aufgrund exothermer Reaktion entstehenden Wärme an einen Wärmeträger in den Rohrpaketen, insbesondere an Wasser/Dampf, wobei die Rohrpakete über eine Ringleitung mit Wasser beaufschlagt und der Dampf über eine Ringleitung abgenommen wird, wobei die Ringleitungen als Sammler oder Kammer (9,10) unmittelbar auf den Reaktormantel (4) aufgesetzt sind,
**dadurch gekennzeichnet,**
**daß** die Verteil- bzw. Sammelkammern (9,9b;10,10b) querschnittlich im wesentlichen kreisförmig ausgebildet sind und sowohl innen als auch außen auf den Reaktormantel aufgesetzt sind, wobei eine Hälfte dem Inneren des Reaktors (4) und die andere Hälfte dem Äußeren des Reaktors zugeordnet ist.

2. Wirbelschicht-Reaktor nach Anspruch 1,
**dadurch gekennzeichnet,**
**daß** die Bohrungen (13) zum Anschluß der Rohrleitungen (7,8) zur Definierung eines gewünschten Druckverlustes und damit zur Vergleichmäßigung der Beaufschlagungen der unterschiedlichen Rohrpakete als Drosselbohrungen ausgeführt sind.

## Claims

1. Fluidised bed reactor (1) for oxychlorination of ethylene, oxygen and HCl with a heat exchanger, comprising several tube packs (2), in the fluidised bed for emitting the heat that arises due to exothermal reaction to a heat exchanger in the tube packs, in particular to water/steam, wherein the tube packs are pressurised by water via a ring conduit and the steam is drawn off via a ring conduit, wherein the ring conduits are placed directly onto the reactor shell (4) as collectors or chambers (9, 10),
**characterised in that**
the distribution or collection chambers (9, 9b; 10, 10b) are designed to be essentially circular in cross section and are placed on the reactor shell both inside and outside, wherein one half is assigned to the interior of the reactor (4) and the other half is assigned to the exterior of the reactor.

2. Fluidised bed reactor in accordance with claim 1,
**characterised in that**
the drilled holes (13) for connecting the pipe conduits (7, 8) are designed as throttling ports for the definition of a desired pressure loss and thus for equalisation of the pressurisations of the different tube packs.

## Revendications

1. Réacteur à lit fluidisé (1) pour oxychloration de l'éthylène, de l'oxygène et du HCl doté d'un échangeur de chaleur constitué de plusieurs faisceaux de tubes (2) dans le lit fluidisé pour délivrer la chaleur produite par réaction exotherme à un caloporteur à l'intérieur des faisceaux de tubes, en particulier à de l'eau/de la vapeur, les faisceaux de tubes étant alimentés en eau par une conduite annulaire et la vapeur étant soutirée par une conduite annulaire, les conduites annulaires étant placées, en tant que collecteurs ou chambres (9, 10) directement sur l'enveloppe du réacteur (4),
**caractérisé en ce que**,
les chambres de répartition, ou collectrices, (9,9 ; 10, 10b) sont une section essentiellement circulaire et **en ce qu'**elles sont placées sur l'enveloppe du réacteur aussi bien à l'intérieur qu'à l'extérieur, une moitié se trouvant à l'intérieur du réacteur (4) et l'autre moitié à l'extérieur du réacteur.

2. Réacteur à lit fluidisé selon revendication 1,
**caractérisé en ce que**
les orifices (13) servant à brancher les tuyauteries (7,8) destinées à définir une perte de pression souhaitée et donc à homogénéiser l'alimentation des différents faisceaux de tubes sont réalisés sous forme d'orifices d'étranglement.
